# EUROPEAN PATENT SPECIFICATION

(11) **EP 1 871 478 B1**
(45) Date of publication and mention of the grant of the patent: **08.06.2016**
(21) Application number: 06813181.2
(22) Date of filing: 12.04.2006
(51) Int. Cl.: A61N 7/00, A61B 17/22

(54) **ULTRASOUND GENERATING APPARATUS**
VORRICHTUNG ZUR ULTRASCHALLERZEUGUNG
APPAREIL DE PRODUCTION D'ULTRASONS

(30) Priority: 12.04.2005 US 103825
(43) Date of publication of application: 02.01.2008
(73) Proprietor: Koninklijke Philips N.V., 5656 AE Eindhoven (NL)
(72) Inventor: SINELNIKOV, Yegor, Port Jefferson, New York 11777 (US); WARNKING, Reinhard, E. Setauket, New York 11733 (US); SARVAZYAN, Armen, Lambertville, NJ 08530-3302 (US); SUTIN, Alexander, Hoboken, NJ 07030 (US)
(74) Representative: Damen, Daniel Martijn
(86) International application number: PCT/US2006/014024
(87) International publication number: WO 2007/013895

(56) References cited:
- EP-A- 1 449 563
- EP-A- 1 449 564
- WO-A-97/47965
- US-A1- 2004 059 265

## Description

### BACKGROUND OF THE INVENTION

The present invention relates to a high intensity ultrasound ablation apparatus utilizing the principle of time-reversed acoustics. Known for years, high intensity focused ultrasound (HIFU) recently became an effective and widespread medical therapy technique. An expected benefit of HIFU is the creation of a clinical effect in a desired, confined location within a body, without damage to intervening tissue.

A broad and diverse range of HIFU therapies, from shock wave lithotripsy, ultrasound enhanced drug deliveries, immune response stimulation, to hemostasis, non-invasive surgery is now in use. See M.R. Bailey et al., 2003, "Physical Mechanisms of the Therapeutic Effect of Ultrasound," Acoustical Physics, 49, 4, pp. 369-388. In HIFU therapy the acoustic field is focused to a target area. Absorption of high intensity ultrasound in a focal region causes a significant temperature rise, resulting in coagulative necrosis of the target tissue. The irreversible ablation within the focal zone is defined by ultrasound source geometry.

In HIFU therapy, it is important to create only target tissue ablation, without damage to other tissue. In certain applications, geometrical focusing of ultrasound to a target is not possible. While current ablation devices and methods produce an ablation pattern which is primarily device dependent, in complex anatomy, an ablation away from a device dependent focal zone is often necessary. It would therefore be desirable for an apparatus to be able to create lesions of variable configuration which are independent of device geometry.

The time reversal principles of ultrasonic wave propagation were first described by Fink, M., 1997, "Time Reversed Acoustics," Physics Today, March 1997, pp. 34-40, which is incorporated herein by reference. Within the range of ultrasonic frequencies, where the adiabatic processes dominate, the acoustic pressure wave propagation equation is time-reversal invariant. This means that for a burst of ultrasound originating from a point in space and later possibly being reflected, refracted or scattered while propagating through the medium toward a transducer, transducer(s) output signals that precisely retrace the propagation path will converge back toward the initial point in space.

Contraction or "beating" of the heart is controlled by electrical impulses generated at nodes within the heart and transmitted along conductive pathways extending within the wall of the heart. Certain diseases of the heart known as cardiac arrhythmias, such as atrial fibrillation, involve abnormal generation or conduction of the electrical impulses. The abnormal conduction routes in atrial fibrillation typically extend from the wall of the heart and along the pulmonary veins of the left atrium. After unwanted electrical impulses are generated in the pulmonary veins or conducted through the pulmonary veins from other sources, they are conducted into the left atrium where they can initiate or continue atrial fibrillation. By deliberately damaging or "ablating" the tissue of the cardiac wall to form a scar along a path crossing the route of abnormal conduction, propagation of unwanted electrical signals from one portion of the heart to another can be blocked.

As described in Fjield et al., U.S. Patent 6,635,054, and in International Publication WO 2004/073505, atrial fibrillation can be treated by ablating tissue in an annular pattern around a pulmonary vein at or around the ostium, the juncture between the pulmonary vein and the heart. As disclosed therein, ablation is performed by making use of high intensity focused ultra-sound. A catheter is introduced into the interior space of the left atrium. The catheter includes a balloon containing an ultrasound reflector collapsed around a cylindrical ultrasound-emitting transducer. When the balloon is inflated, the reflector assumes a shape that focuses the ultrasonic energy emitted by the transducer in a ring-like pattern on the cardiac tissue at the ostium, producing an annular scar.

Although the *Fjield* '054 system produces a scar at the desired location, the size and shape of the ultrasound pattern is determined by the configuration of the reflector. This limits to some degree the size and shape of the scar that can be produced and the ability of the physician to adapt the treatment to variations in the anatomy of patients. In many cases, for example, to avoid phrenic nerve damage, physicians may need to exclude a certain region from application of ultrasound. Also, variability in ostium size requires catheter exchanges. Thus, flexibility with respect to the lesion shape and size produced by an ablation method and apparatus would be desirable to address varying anatomical situations.

Another technique for performing cardiac ablation is disclosed in Govari et al., U.S. Patent Application Publication No. 2004/0162550. An unfocused ultrasound emitter (a "beacon") is introduced to a target site inside the heart through a catheter. Several duplex (emitter and detector) ultrasound transducers are placed outside the body in the vicinity of the heart, and the beacon is activated. The ultrasound originating from the beacon is sensed by the external duplex transducers, and the signals they produce are reversed in time, and each such signal is used to drive the respective transducer into emission. As disclosed in Fink U.S. Pat. No. 5,431,053, the ultrasound signals produced by the external duplex transducers will combine to produce a focused spot of ultrasound energy at the site of the beacon. By moving around the beacon and repeating the sensing/emitting operation of the external duplex transducers, it becomes possible to produce an ablation in any desired pattern. Although it is possible for a surgeon to produce any desired shape of scar by moving around the beacon, this is a very demanding and cumbersome process.
EP-A-1449564 discloses an apparatus for performing ablation of cardiac tissue using time-reversed ultrasound signals, in accordance with the preamble of claim 1. An apparatus is provided for performing ablation of cardiac tissue using ultrasound.
EP-A-1449563 relates to an externally applied high-intensity focused ultrasound (page IFU) for therapeutic treatment. An apparatus for performing the therapeutic procedure using ultrasound is provided, including a beacon adapted to be placed at a site in a body of a subject and a set of one or more ultrasound transducers, each transducer adapted to detect a respective beacon signal coming from the beacon, and adapted to output a time-reversed ultrasound signal, reversed in time with respect to a property of at least one of the beacon signals.

### SUMMARY OF THE INVENTION

The invention provides for an ultrasonic energy application apparatus in the independent claim.

In accordance with one aspect of the present invention, ultrasonic transducers in a predefined arrangement are maintained in ultrasonic communication with the body and are operated using actuating signals derived from pre-existing, stored representations of sensor signals which would be detected by the transducers in response to ultrasound energy, such as a brief ultrasonic impulse, originating from an emitter at a number of predetermined points constituting a pattern. The actuating signals most preferably constitute a time-reversed replica of the sensor signals, so that the ultrasonic signal emitted by the transducers substantially recreates the original ultrasonic signal at the points constituting the pattern. By placing the transducers in a predetermined spatial relationship, the ablation pattern may be formed in a desired location within the body. For example, by placing the set of transducers in a known spatial relationship to the heart, the ablation pattern may be formed around the ostium of a pulmonary artery.

In one embodiment, the stored representations may be derived by placing a reference source and the set of ultrasonic transducers, while in their predefined arrangement, in a medium having ultrasonic properties approximating that of the environment in which ablation is to be performed as, for example, a medium such as an aqueous medium having ultrasonic properties approximating that of blood and muscle. The reference source is actuated to emit ultrasonic energy while the reference source is disposed at a point having known disposition in the frame of reference of the set of transducers. Where the set of transducers includes a plurality of transducers, each transducer produces a sensor signal component representing ultrasound energy sensed by it, and the sensor signal components of the various transducers, or time-reversed versions of the sensor signal components, are stored as a representation of the sensor signal associated with the point. This process is replicated for other points producing corresponding sensor signals.

Because the representations of the sensor signals are available before the transducers are placed on or in the body, there is no need to place a beacon within the body where ablation is desired, and no need to trace the pattern to be ablated by moving such a beacon within the body.

Representations of sensor signals can be obtained for numerous points in a two-dimensional or three-dimensional grid, with each point defined in the frame of reference of the transducers, so as to provide a group of stored representations, each associated with a grid point in the frame of reference of the transducers. Such a group of stored representations may be used to form a pattern approximating virtually any shape within the range encompassed by the grid.

In a further aspect, the representations of the sensor signals can be derived from signals sensed by a model of the set of transducers. For example, where numerous probes, all having substantially identical sets of transducers, are manufactured, one such probe can be used as a model to derive the sensor signals. In other aspects, the sensor signals can be derived using the set of transducers, or a model of the set of transducers in the medium as discussed above, to emit ultrasonic energy, while receiving the signal using a receiver disposed at a point. For example, where the set of transducers, or the model of the set of transducers, includes multiple transducers, each transducer can be driven individually with a signal approximating an impulse while the other and the signal received at the point can be recorded as a signal component. Due to reciprocity, this signal component will be the same as a signal component received by the same transducer if an emitter disposed at the point was actuated by the same impulse signal.

A further aspect of the invention provides ultrasonic energy application apparatus. Apparatus according to this aspect of the invention desirably includes a set of ultrasound transducers including one transducer or a plurality of transducers arranged in predefined locations relative to one another. The apparatus most preferably includes a source of a plurality of pre-existing representations of sensor signals, each such sensor signal corresponding to a signal that would be sensed by the ultrasound transducers if an ultrasound emitter were placed in a particular point in the frame of reference of the transducers. The plurality of representations of sensor signals thus include different representations associated with each of a group of points in the frame of reference of the transducers. For example, each stored representation may be a sensor signal or a time reversal of a sensor signal, and may include multiple components, each associated with an individual transducer in a set of transducers including a plurality of transducers. The apparatus desirably also includes an actuator for driving transducers in response to said pre-existing representations with actuating signals corresponding to time reversals of said sensor signals.

### BRIEF DESCRIPTION OF THE DRAWINGS

The foregoing brief description, as well as further objects, features and advantages of the present invention will be understood more completely from the following detailed description of certain aspects, with a reference being had to the accompanying drawings, in which:
Fig. 1 is a schematic representation of a probe according to one embodiment of the present invention;
Fig. 2 a schematic representation of a transducer assembly included in the probe of Fig. 1;
Fig. 3 is a schematic diagram useful in explaining certain principles of time reversed acoustics as applied in a method according to one aspect of the invention using the probe of Fig. 1.;
Fig. 4 is a functional block diagram illustrating an apparatus incorporating the probe of Fig. 1 during ultrasonic ablation in accordance with one embodiment of the present invention.
Fig. 5 is a diagram similar to Fig. 3 depicting one step in a method according to a further aspect of the invention using the probe of Fig. 1.
Figs. 6 and 7 are diagrams similar to Fig. 1 but depicting a probe assembly according to further embodiments of the invention.

### DETAILED DESCRIPTION

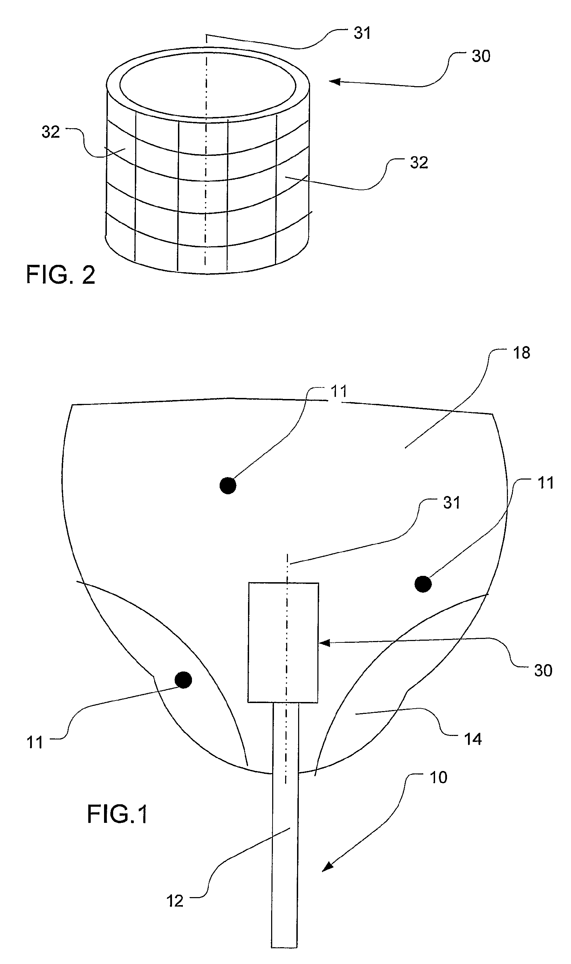

Turning now to the details of the drawings, Fig. 1 is a schematic representation of an embodiment of a probe in accordance with the present invention. A probe 10 includes a catheter 12 having a distal end bearing an outer, reflector balloon 14; an inner, structural balloon 18; and a transducer subassembly 30. *Fjield* '054 and International Publication WO 2004/073505, discussed above, disclose in more detail various probe structures of this type. Such disclosure is incorporated herein by reference.

Prior to use, the probe would be in a collapsed state, in which both balloons are collapsed about the transducer subassembly 30. Preferably, this probe is for use in cardiac ablation. Accordingly, it could be inserted over a guide wire, through a sheath which, in accordance with conventional practice, has previously been threaded through a patient's circulatory system and into the left atrium of the heart. However, there are other known techniques for positioning the probe, including surgical procedures.

Following that, the structural balloon 18 may be inflated by injecting through a lumen of the catheter 12 a liquid, such as saline solution, which has an ultrasonic impedance approximating that of blood. The reflector balloon 14 is inflated by injecting through another lumen of catheter 12 a gas, such as carbon dioxide. Owing to the different ultrasound impedance of the two inflation media, the interface between balloons 14 and 18 would then form a forwardly-facing reflecting surface adapted to reflect ultrasound waves forward, through the distal portion of the balloon 18.

Probe 10 also includes one or more position-determining elements 11 which lie in a predetermined spatial relationship to the transducer assembly 30. These position-determining elements are arranged so that the disposition of the position-determining elements, and hence the disposition of the transducer assembly including its position and orientation, can be detected during use of the probe. In Fig. 1, these are depicted as a set of three point markers such as radio-opaque markers which can be visualized using x-ray or fluoroscopic imaging. Other point markers suitable for magnetic resonance imaging may be used.

Alternatively or additionally, the position-determining elements may include magnetic or electromagnetic transducers which can interact with external magnetic or electromagnetic transducers to determine the position or orientation of a probe in the frame of reference of these external devices. Such transducer systems are well known in the art.

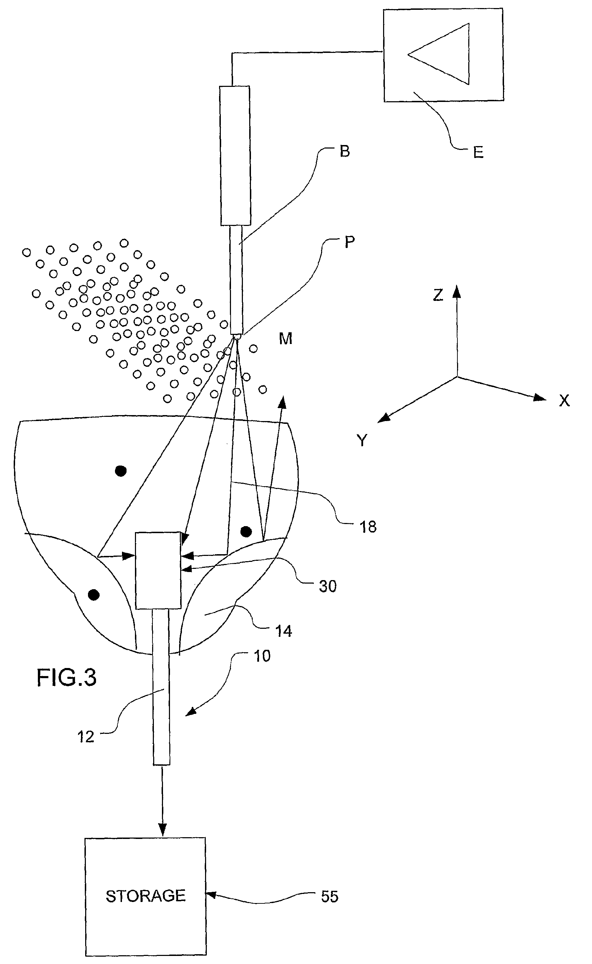

Fig. 2 is a schematic representation of the transducer assembly 30. In this embodiment, it is cylindrically shaped and includes a set transducers, in this case a plurality of ultrasound transducers 32 which cover its surface. The individual transducers 32 may be physically separate elements or may be formed as a unitary body of piezoelectric material, such as the hollow tubular body depicted in Fig. 3, with individual ground or signal electrodes covering different portions of the unitary body so that each portion acts partially or completely independently of the other portions and hence constitutes a separate transducer. In this particular embodiment, the transducer subassembly 30 or set of transducers, and the reflective surface defined by the balloons 14 and 18 in their inflated condition are substantially symmetrical about a common axis 31. The assembly may be virtually any other shape.

When exposed to ultrasonic energy, each of the transducers 32 independently senses ultrasonic energy incident upon it, producing a time varying signal on conductors (not shown) within probe 10 associated with that transducer. That sensor signal represents the ultrasound impinging on the individual transducers. Each of the transducers will also emit ultrasound energy when actuated by an electrical signal provided via the same conductors .

Fig. 3 is a schematic diagram useful in explaining the principle of time reversed acoustics as it applies in this embodiment. The probe 10, with the balloons 14 and 18 inflated as discussed above, and a reference ultrasound source such as an emitter or beacon B are both present in a medium M. Medium M desirably has acoustic properties, such as acoustic velocity and acoustic impedance, simulating the acoustic properties of the environment in which the balloons and transducer assembly will be placed when performing ablation. For example, where the balloons and transducer assembly will be disposed within the heart of the body, the medium may be water to simulate the acoustic properties of blood. In the particular embodiment shown, the medium has uniform and substantially isotropic acoustic properties.

The beacon is disposed at a point P within the frame of reference of the transducer assembly and balloons. This frame of reference is schematically indicated by Cartesian coordinates x, y, z in Fig. 3. Any other coordinate system, such as polar or cylindrical coordinates, may be used. The beacon is actuated by an electrical signal source E, desirably with a signal approximating an impulse. The beacon produces ultrasound energy which travels in all directions.

Ultrasound entering the structural balloon 18 will either impinge directly upon transducer assembly 30, or it will be reflected one or more times from the interface between the two balloons and either exit the probe or impinge upon the transducer subassembly 30. The ultrasound energy impinging upon the transducer subassembly 30 will be sensed by one or more of the transducers 32.Each transducer 32 will produce a time-varying electrical sensor signal component representing the ultrasound energy it senses. If these sensor signal components were reversed in time and used to actuate their respective sensors, the signals thus produced would cooperatively reproduce at point P the signal originally produced at point P by the beacon B.

A representation of the plural signal components is stored in a storage device 55 in any convenient form and associated with the particular point P. The storage process may include conventional signal processing such as amplification and digitization using conventional elements (not shown). For example, each component may be stored as an analog or digital record of the component as originally received by a particular transducer 32, or as a corresponding record of the same signal with the time scale reversed. A digital record may include a series of values each representing a sample of the component waveform at a particular time. Such a series may be read out of storage in the original order, or may be read out in reverse order to provide a time-reversed representation. Thus, in this embodiment, the representation associated with a particular point P will include a plurality of components, each associated with a particular transducer. The representation as a whole represents the sensor signal received by the entire set of transducers in subassembly 30.

The same process is repeated with beacon B at a plurality of points 115 constituting a two-dimensional or, more preferably, three-dimensional grid of points, so that a representation of the sensor signals is stored for each of the plural points, each such representation being associated with a particular point defined in the frame of reference of the transducer assembly. The grid of points need not be a rectilinear grid; it may include concentric circular arrays of points, or points at irregularly spaced locations.

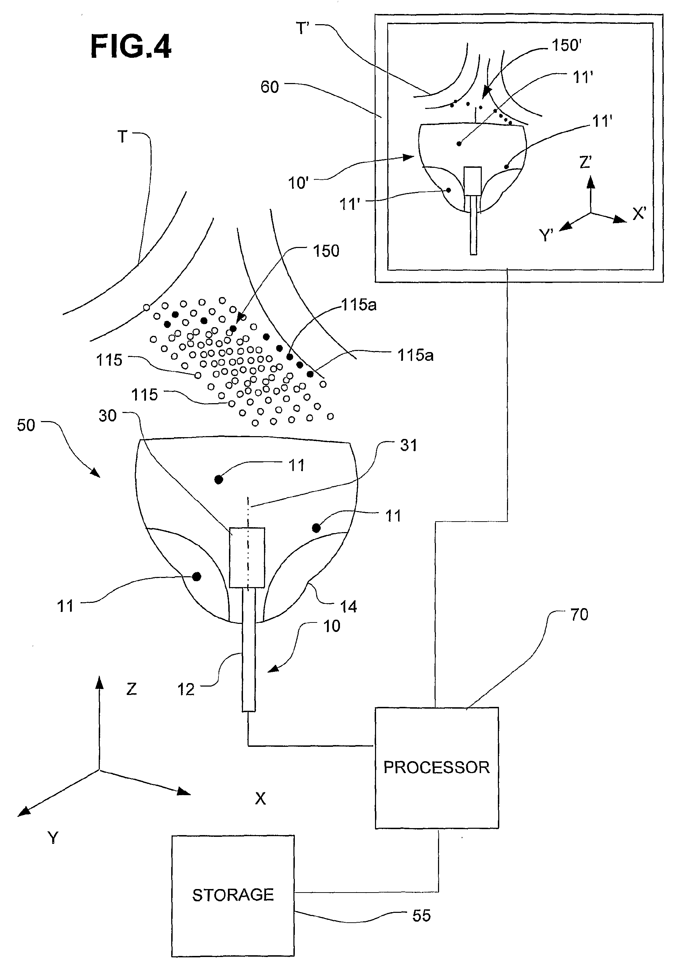

Fig. 4 is a functional block diagram illustrating the operation of a preferred apparatus 50 incorporating probe 10 to perform ultrasound ablation. Apparatus 50 includes a probe 10 of the type already described, a storage unit 55 holding the representations of sensor signals as discussed above, a display 60, and a processor 70. Processor 70 may include the elements of a conventional general-purpose digital computer, and may also include digital-to-analog conversion circuitry and amplification circuitry for providing actuation signals as discussed below. Prior to and during treatment, the patient's heart and the probe 10 may be observed through a fluoroscope, a CAT, or any other conventional imaging device, with the image being displayed on display 60. This permits the surgeon to plan how ablation will be performed. With probe 10 positioned within the heart as explained above, the surgeon can assure the rotational position of the probe by bringing index marks 11 to a reference position relative to the structures of the patient's body, at or near the prospective location of an ablation, so that the ultrasonic transducer assembly is in ultrasonic communication with tissues at such location. Catheter 12 also may include steering elements which can be used to tilt the distal end of the catheter, and hence tilt the transducer subassembly 30 and the reflective surface defined by the balloons.

The operator may then select the shape, size and rotational orientation of the desired ablation pattern, in the frame of reference of the probe, such as by selecting from a menu of standard patterns stored in processor 70 or in storage unit 55.

The operator may also draw a pattern with a light pen or a mouse superimposed on the image displayed on screen 60. An internal calculation will then determine the best fit between selected pattern and stored ablation points. This is of particular importance in certain anatomical situations where cavities or vessels are in close vicinity leaving only a small tissue ridge to be ablated. An example is the left pulmonary veins and the left atrial appendage lying closely together leaving only a small tissue ridge between them.

Such selection may be based upon knowledge of the position of the probe and transducer assembly relative to the body tissues to be ablated. For example, if the probe is positioned so that the distal face of balloon 18 confronts a wall of the heart with the axis of the transducer assembly and probe aligned with the axis of a pulmonary vein, the physician may select a stored pattern in the form of a ring or loop of specified diameter encircling the axis in a plane just distal to such distal face.

Once the desired ablation pattern has been selected, processor 70 identifies those points 115a (shown shaded in Fig. 4) from among the grid points 115, in the frame of reference of the probe, which constitute the pattern. The processor then selects a stored signal representation from storage unit 55 associated with a first identified one of the points 115a, and generates actuation signals based on the stored signal representation corresponding to a time-reversed replica of the sensor signals which were produced by the various transducers in response to ultrasound emitted from that point. For example, if the stored signal representations include series of digital samples of the originally-received sensor signals, the processor may simply read out the samples constituting the signal component for each transducer in reverse order and convert the resulting digital signal to analog form to create an actuation signal component for the corresponding transducer. The processor applies the actuation signal components simultaneously to a plurality of the transducers, and preferably to all of the transducers. The resulting ultrasonic emissions from the transducer assembly create a replica of the ultrasonic impulse at the point, and thus cause micro cavitation at such point. The same process is then repeated for the other points in the pattern. In a variant, impulses can be provided at two or more points simultaneously by applying actuation signals based on the stored representations associated with two or more points simultaneously.

The application of time reversed signals can be combined with a standard ultrasound therapy procedure that can be executed by the same transducers. The effect of micro cavitation will enhance ultrasound absorption and tissue impact at the site of time reversed signal convergence, i.e., at locations on the pattern where cavitation has been induced by the impulses. Continuous ultrasound signal can be delivered following a single or a series of time reversed impulses.

Signals can be obtained in a laboratory setting to create time reversed signals that would affect a volume of tissue rather than discrete points. A collection of applicable shape transducers can be used to generate the reference signal, which is sensed by all probe transducers and reversed in time and recorded. Subsequently, the probe transducers can be actuated with respective recorded signals, to create a simultaneous tissue impact at a volume of tissue directly corresponding to shape transducers. A cavitation cloud can be generate this way substantially simultaneously over a volume of the tissue, and it can be controlled by repetitive application of the same set of signals.

It also can be combined with continuous wave ultrasound delivery between time reversed pulses to enhance ultrasound absorption due to cavitation and to keep cavitational bubbles from collapsing. Also, the actuation signals associated with each point or volume may be applied repeatedly with varying amplitude parameters.

In a further variant, the stored representations may include only representations associated with points constituting a single pattern as, for example, a ring of a particular diameter at a particular location in the frame of reference of the probe and transducer assembly. In this case, the physician maneuvers the probe to a predetermined position to properly align the pattern with the body tissues, and then instructs the processor to begin ablation. The processor forms and applies the actuation signals corresponding to each of the stored representations.

In yet a further variant, the processor determines the disposition of the probe, and hence the transducer assembly, relative to the body of the patient and specifies the points constituting a pattern so that the pattern lies in the desired location within the patient's body. As shown in Fig. 4, the data constituting an image of the relevant portion of the patient (the tissues T of the heart wall) is supplied to the processor and the processor generates an image on display 60 representing this portion of the patient in an image frame of reference. The processor is also supplied with data specifying the disposition of the probe in the image frame of reference. For example, using conventional input devices (not shown) connected to processor 70, the physician may move a cursor on display 60 into alignment with the image 11' of each of the spot markers 11 on probe 10, and inputs a signal to the processor when such alignment is achieved. Repeating this process using images in two orthogonal planes completely specifies the location of the spot markers in the image frame of reference.

In as much as the locations of these markers in the frame of reference of the probe is known, this fully specifies the disposition of the probe in the image frame of reference, and provides all of the information necessary to derive a geometric transformation between the image frame of reference (and hence the frame of reference of the patient's body) and the frame of reference of the probe.

In a system where the position-determining elements of the probe include magnetic or electromagnetic transducers, the information specifying the disposition of the probe may be acquired in a frame of reference associated with the transducers, and transformed into the image frame of reference using data relating the transducer frame of reference to the image frame of reference.

The physician may specify the desired ablation pattern directly in image frame of reference by drawing the desired pattern on the screen, using conventional computer input devices. For example, the screen may be a touch-sensitive screen. Computer techniques for selecting and drawing shapes are well known in the art. The desired pattern is then transformed into the frame of reference of the probe. Processor 70 is then operated to select those points which lie on the desired pattern. For example, in Fig. 4, the desired pattern has been traced as a curve 150' in the image frame of reference X'Y'Z' on display 60'. This curve is transformed into a theoretical curve 150 in the XYZ frame of reference of the probe 10. Points 115ashown in solid black lie on this curve, or in close proximity to it. The processor 70 selects these points as the points constituting the pattern. In the same manner as discussed above, the processor 70 creates a set of actuation signals for transducer assembly 30 that will produce the ablation pattern.

In a variant of this approach, the step of determining the disposition of the probe relative to the patient's body may be repeated during the step of applying the actuation signals. For example, the determining step may be repeated after each point is treated. If the disposition changes, the transformation between the frame of reference of the body and the frame of reference of the probe will also change, and processor 70 therefore will select a new set of points constituting the untreated portions of the desired pattern. This avoids the need to hold the probe at a constant location relative to the patient's body during the entire ablation step and compensates for cardiac motion or breathing artifacts.

Memory storage element 55 is depicted as an element separate from processor 70 and separate from probe 10. However, if probe 10 is the only probe specified for use with the processor, the information may be contained in a ROM (read only memory) chip or other element of the processor. Alternately, the processor may be designed for use with different probes and, during setup, the probe being used is specified, automatically designating a special section of storage to be accessed for the transducer drive signal information.

In a further variant, a physical element such as a semiconductor chip or other data storage medium 55 may be incorporated in probe 10 or supplied with the probe in a kit. In a further variant, the storage element may be at a remote location accessible to processor 70 via the Internet or other communications link. For example, the probe manufacturer may maintain sets of stored signal representations appropriate for various probes.

In any case, the processor will have access to storage containing the necessary information to generate a set of actuation signals that will drive the transducers of the probe so to produce the patterns discussed above.

In the signal representation storing process as described above with reference to Fig. 3, the same probe 10 and transducer assembly 30 which is used to perform the ablation is also used to acquire the sensor signals. However, this is not essential. A different probe and transducer assembly referred to herein as a "model" of the transducer assembly used for ablation, can be used, provided that the model accurately reflects the characteristics such as signal response of the actual transducer assembly used to perform the ablation. Where the actual probe includes a reflector, the model desirably includes an identical reflector. Where a model is used it would never be necessary to use the transducers 32 of the transducer assembly 30 used for the ablation as ultrasound sensors. These transducers serve only as ultrasound emitters. Where numerous probes and transducer assemblies are mass-produced, one such device can serve as a model, and the others can be used for ablation.

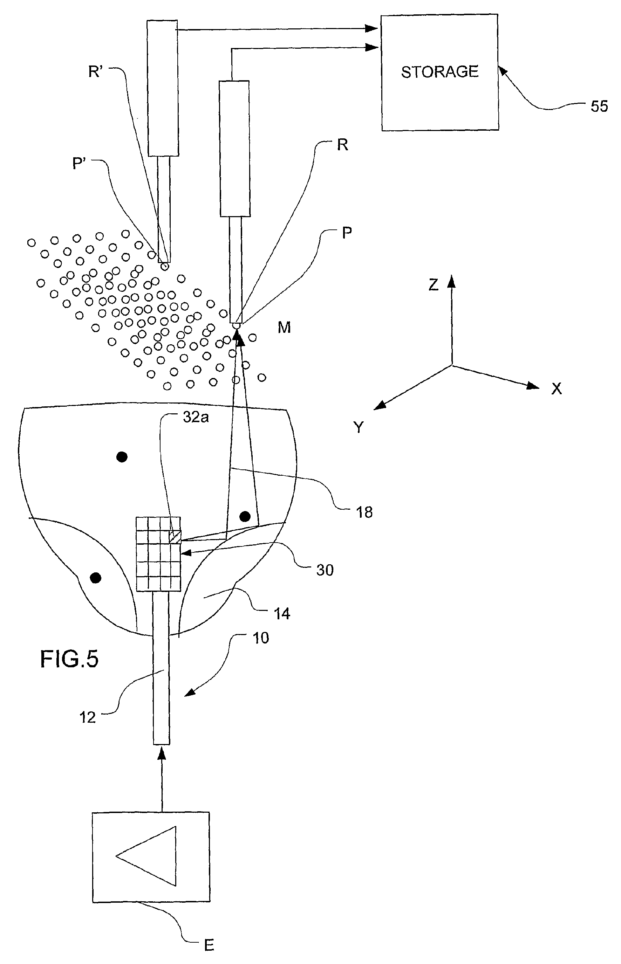

Another method of acquiring the signal representations to be used as stored signal representations is schematically depicted in Fig. 5. In this method, the set of transducers 30 and the inflated reflector balloon 14 and structural balloon 18 are again immersed in a medium such as that discussed above which simulates the acoustic properties of the body tissues. An ultrasonic receiver R which is adapted to sense ultrasound at a sensing point is also disposed in the medium. While the receiver R is disposed so that its sensing point is at a known point P in the geometric frame of reference of the transducer set, a signal source E actuates one transducer 32a of the transducer set 30, desirably using a signal approximating an impulse. Due to reciprocity, the ultrasonic signals impinging on the receiver R will be identical to the ultrasonic signals which would impinge on transducer 32a if an impulse was emitted at point P. The electrical signals generated by receiver R thus constitute a signal component identical (apart from amplitude scaling) to those which are generated by transducer 32a when an impulse is emitted at point P, as in the method discussed above with reference to Fig. 3. Thus, in the aspect of Fig. 5, the electrical signal generated by receiver R constitutes a signal component which corresponds to the ultrasonic signal which would be received by transducer 32a. A representation of this signal component is stored in storage element 55 as the component associated with point P and with transducer 32a. The same process is repeated for all of the other transducers in the set of transducers, and for all of other points in a grid of points. Thus, at the end of the process, storage element 55 contains a representation of a sensor signal associated with each point P, each such representation including plural components associated with individual transducers. These stored representations are used in exactly the same way as discussed above. This approach offers certain practical advantages. For example, it is not necessary to use the transducers of set 30 as receivers. Also, it is generally easier to provide a receiver sensitive at a point than an emitter operative to emit from a point. A set of plural receivers may be provided at multiple points simultaneously. Thus, in Fig. 5, another receiver R' detects ultrasound at point P' at the same time as receiver R detects ultrasound at point P. The detected signals provide signal components associated with point P', and representations of these components are also stored in storage element 55.

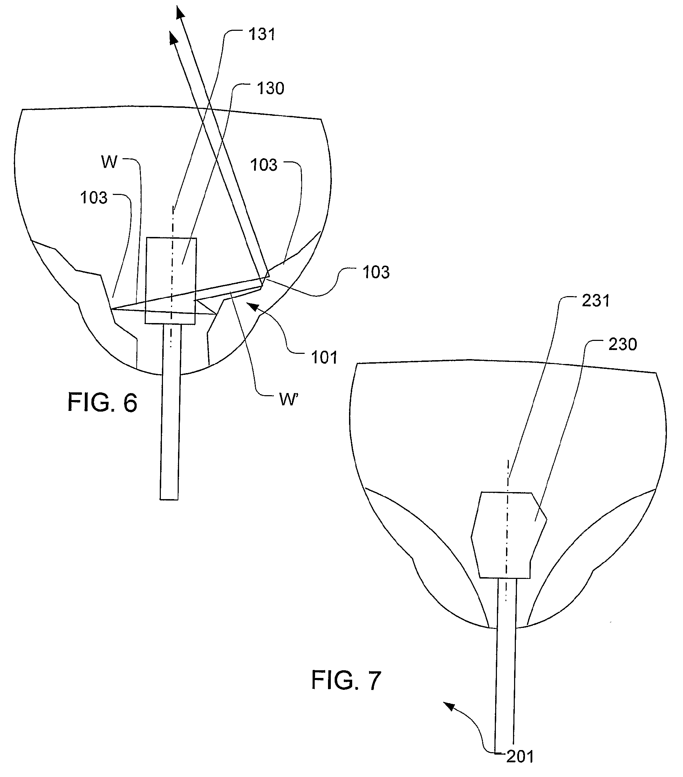

In a further embodiment (Fig. 6), the set of transducers 130 includes a single cylindrical transducer symmetrical about an axis 131. A reflector structure, such as a balloon reflector structure, defines a reflective surface 101 which incorporates numerous individual sections having different spatial orientations. The reflective surface as a whole is not symmetrical about axis 131. This structure can be used in substantially the same manner as discussed above. However, with only a single transducer, the stored representation of a sensor signal associated with each point will include only a single component. In yet another variant, the reflective surface may be axially symmetric but non-coaxial with the transducer set. As also shown in Fig. 6, the reflector, whether symmetrical or assymetrical, may provide reverberation. Thus, in Fig. 6, certain ultrasound waves W are reflected from one part of the reflector to another before exiting from the balloon structure. Waves W and thus take a substantially longer path than ultrasound waves W', which are reflected only once. This diversity in path length is desirable.

Apparatus according to yet another variant (Fig. 7) includes a transducer set 230, which may be a single transducer or a set including plural transducers, as well as a reflector structure defining a reflective surface 201 which is symmetrical about axis 231. The transducer set 230, however, is not symmetrical about axis 231.

It will be appreciated that the use of the disclosure for cardiac ablation is merely an exemplary application, as the disclosure should find broad application in surgical and non-surgical treatments.

It is not essential to provide a reflector associated with the transducer unit. For example, the transducer set may include a planar or other array of transducers having emitting surfaces facing generally in a forward direction. Also, the probe and other aspects of the invention are not limited to use inside a living body. For example a probe could be positioned outside the body so as to inject ultrasound energy to a specific location within the body, for example to perform ablation, provide localized heating or destroy a kidney stone.

Although a preferred embodiment of the invention has been disclosed for illustrative purposes, those skilled in the art will appreciate that many additions, modifications and substitutions are possible without departing from the scope of the invention as defined by the accompanying claims.

## Claims

1. A system comprising an ultrasonic energy application apparatus operable for applying a set of one or more actuating signals to a set of first ultrasound transducers (32) and a model, the set of first ultrasound transducers including a plurality of first transducers in predefined locations relative to one another in ultrasonic communication with a body, the actuating signals corresponding to a time-reversed version of sensor signals representing the ultrasound signals that would be detected by the set of first transducers while in their predefined locations if an ultrasound emitter (B) were placed in a set of plural positions defining a pattern in a frame of reference defined by said set of first transducers, wherein the ultrasonic energy application apparatus is operable for the application of the actuating signals by using representations of the sensor signals obtained by
(a) providing a set of stored signals associated with a grid of points (115) in said frame of reference, each said stored signal corresponding to a time reversal of sensor signals representing one or more ultrasound signals that would be detected by the set of first transducers if an ultrasound emitter were placed at a point associated with such stored signals; and
(b) selecting for application to the first transducers only those of the stored signals associated with points lying on the pattern,
**characterized in that**
the system further comprises a reference source, wherein the stored sensor signals are obtained by placing the reference source and the model having one or more receivers at one or more locations corresponding to one or more locations of the first transducers in said set in a reference medium emulating the ultrasound response of the body and actuating said reference source to emit ultrasonic energy from model points in the frame of reference of said model corresponding to said points in said frame of reference of said first transducers,
or wherein the system further comprises a receiver (B), wherein the stored sensor signals are obtained by placing the receiver and the model having a set of second transmitters including one or more second transmitters at one or more locations corresponding to one or more locations of the first transducers in said set in a reference medium emulating the ultrasound response of the body and actuating each second transmitter in said set of second transmitters to emit ultrasonic energy while said receiver is disposed at model points in the frame of reference of said model corresponding to said points in said frame of reference of said first transducers.

2. The system of claim 1, wherein said actuating signals are constituted so that the pattern is at least two-dimensional.

3. The system of claim 2, wherein said set of first transducers includes a plurality of transducers and each of said stored signals includes a plurality of components, each such component being associated with one of said transducers, said step of actuating each transducer in said set being performed by actuating each of the transducers in said set individually while said receiver is disposed at each of said points.

4. The system of claim 1, wherein said set of transducers includes a plurality of first transducers and each of said stored signals includes a plurality of components, each such component being associated with one of said transducers, said step of actuating each transmitter in said set of transmitters being performed by actuating each of the transmitters in said set individually while said receiver is disposed at each of said model points.

## Patentansprüche

1. System umfassend ein Ultraschallenergie-Zuführungsgerät, das betriebsfähig ist, um eine Gruppe von einem oder mehreren betätigenden Signalen einer Gruppe von ersten Ultraschallwandlern (32) zuzuführen, und ein Modell,
wobei die Gruppe von ersten Ultraschallwandlern eine Vielzahl von ersten Wandlern an vordefinierten Orten in Bezug zueinander in Ultraschallkommunikation mit einem Körper umfasst, die betätigenden Signale einer zeitlich-umgekehrten Version von Sensorsignalen entsprechen, die die Ultraschallsignale darstellen, welche durch die Gruppe von ersten Wandlern an ihrer vordefinierten Orten detektiert würden, wenn ein Ultraschallemitter (B) an einer Gruppe von mehreren Orten platziert wäre, die ein Muster in einem durch die genannte Gruppe aus ersten Wandlern definierten Referenzrahmen definieren, wobei das Ultraschallenergie-Zuführungsgerät betriebsfähig ist für die Zuführung der betätigenden Signale durch Verwenden von Darstellungen der Sensorsignale, die erlangt wurden durch
(a) Bereitstellen einer Gruppe von gespeicherten Signalen, die einem Raster aus Punkten (115) in dem genannten Referenzrahmen zugehörig sind, wobei jedes gespeicherte Signal einer zeitlichen Umkehrung von Sensorsignalen entspricht, die ein oder mehrere Ultraschallsignale darstellen, welche durch die Gruppe von ersten Wandlern detektiert würden, wenn ein Ultraschallemitter an einem zu diesen gespeicherten Signalen gehörigen Punkt platziert würde; und
(b) Auswählen von nur denjenigen der gespeicherten Signale, die zu Punkten auf dem Muster gehören, zur Zuführung zu den ersten Wandlern,
**dadurch gekennzeichnet, dass**
das System weiterhin eine Referenzquelle umfasst, wobei die gespeicherten Sensorsignale erlangt werden, indem die Referenzquelle und das Modell mit einem oder mehreren Empfängern an einem oder mehreren Orten platziert werden, die einem oder mehreren Orten der ersten Wandler in der genannten Gruppe in einem Referenzmedium entsprechen, das die Ultraschallreaktion des Körpers emuliert, und die genannte Referenzquelle betätigt wird, um Ultraschallenergie von Modellpunkten in dem Referenzrahmen des genannten Modells zu emittieren, die den genannten Punkten in dem genannten Referenzrahmen der genannten ersten Wandler entsprechen,
oder wobei das System weiterhin einen Empfänger (B) umfasst, wobei die gespeicherten Sensorsignale erlangt werden, indem der Empfänger und das Modell mit einer Gruppe von zweiten Sendern, einschließlich einem oder mehreren zweiten Sendern, an einem oder mehreren Orten platziert werden, die einem oder mehreren Orte der ersten Wandler in der genannten Gruppe in einem Referenzmedium entsprechen, das die Ultraschallreaktion des Körpers emuliert, und jeder zweite Sender in der genannten Gruppe der zweiten Sender betätigt wird, um Ultraschallenergie zu emittieren, während der genannte Empfänger an Modellpunkten im Referenzrahmen des genannten Modells angeordnet ist, die den genannten Punkten in dem genannten Referenzrahmen der genannten ersten Wandler entsprechen.

2. System nach Anspruch 1, wobei die genannten betätigenden Signale so zusammengesetzt sind, dass das Muster zumindest zweidimensional ist.

3. System nach Anspruch 2, wobei die genannte Gruppe von ersten Wandlern eine Vielzahl von Wandlern umfasst und wobei jedes der genannten gespeicherten Signale eine Vielzahl von Komponenten umfasst, wobei jede dieser Komponenten zu einem der genannten Wandler gehört, wobei der genannte Schritt des Betätigens jedes Wandlers in der genannten Gruppe durchgeführt wird, indem jeder der Wandler in der genannten Gruppe einzeln betätigt wird, während der genannte Empfänger an jedem der genanten Punkte angeordnet ist.

4. System nach Anspruch 1, wobei die genannte Gruppe von Wandlern eine Vielzahl von ersten Wandlern umfasst und jedes der genannten gespeicherten Signale eine Vielzahl von Komponenten umfasst, wobei jede dieser Komponenten zu einem der genannten Wandler gehört, wobei der genannte Schritt des Betätigens jedes Wandlers in der genannten Gruppe von Wandlern durchgeführt wird, indem jeder der Wandler in der genannten Gruppe einzeln betätigt wird, während der genannte Empfänger an jedem der genannten Modellpunkte angeordnet ist.

## Revendications

1. Système comprenant un appareil d'application d'énergie ultrasonore exploitable pour appliquer un ensemble d'un ou plusieurs signaux d'actionnement à un ensemble de premiers transducteurs à ultrasons (32) et un modèle,
l'ensemble de premiers transducteurs à ultrasons comprenant une pluralité de premiers transducteurs dans des emplacements prédéfinis les uns par rapport aux autres en communication ultrasonore avec un corps, les signaux d'actionnement correspondant à une version inversée dans le temps de signaux de capteur représentant les signaux ultrasonores qui seraient détectés par l'ensemble de premiers transducteurs alors qu'ils se trouvent dans leurs emplacements prédéfinis si un émetteur d'ultrasons (B) était placé dans un ensemble de positions multiples définissant un motif dans un cadre de référence défini par ledit ensemble de premiers transducteurs, dans lequel l'appareil d'application d'énergie ultrasonore est exploitable pour l'application des signaux d'actionnement en utilisant des représentations des signaux de capteur obtenues par
(a) la fourniture d'un ensemble de signaux stockés associés à une grille de points (115) dans ledit cadre de référence, chaque signal stocké précité correspondant à une inversion dans le temps de signaux de capteur représentant un ou plusieurs signaux ultrasonores qui seraient détectés par l'ensemble de premiers transducteurs si un émetteur d'ultrasons était placé à un point associé à de tels signaux stockés ; et
(b) la sélection pour l'application aux premiers transducteurs uniquement de ces signaux parmi les signaux stockés, associés à des points se trouvant sur le motif,
**caractérisé en ce que** le système comprend en outre une source de référence, dans lequel les signaux de capteur stockés sont obtenus en plaçant la source de référence et le modèle possédant un ou plusieurs récepteurs à un ou plusieurs emplacements correspondant à un ou plusieurs emplacements des premiers transducteurs dans ledit ensemble dans un milieu de référence émulant la réponse aux ultrasons du corps et en actionnant ladite source de référence pour émettre de l'énergie ultrasonore à partir de points de modèle dans le cadre de référence dudit modèle correspondant auxdits points dans ledit cadre de référence desdits premiers transducteurs,
ou **caractérisé en ce que** le système comprend en outre un récepteur (B), dans lequel les signaux de capteur stockés sont obtenus en plaçant le récepteur et le modèle possédant un ensemble de seconds émetteurs comprenant un ou plusieurs seconds émetteurs à un ou plusieurs emplacements correspondant à un ou plusieurs emplacements des premiers transducteurs dans ledit ensemble dans un milieu de référence émulant la réponse aux ultrasons du corps et en actionnant chaque second émetteur dans ledit ensemble de seconds émetteurs pour émettre de l'énergie ultrasonore alors que ledit récepteur est disposé à des points de modèle dans le cadre de référence dudit modèle correspondant auxdits points dans ledit cadre de référence desdits premiers transducteurs.

2. Système selon la revendication 1, dans lequel lesdits signaux d'actionnement sont constitués de sorte que le motif soit au moins bidimensionnel.

3. Système selon la revendication 2, dans lequel ledit ensemble de premiers transducteurs comprend une pluralité de transducteurs et chacun desdits signaux stockés comprend une pluralité de composants, chaque composant tel étant associé à un desdits transducteurs, ladite étape d'actionnement de chaque transducteur dans ledit ensemble étant exécutée par l'actionnement de chacun des transducteurs dans ledit ensemble individuellement alors que ledit récepteur est disposé à chacun desdits points.

4. Système selon la revendication 1, dans lequel ledit ensemble de transducteurs comprend une pluralité de premiers transducteurs et chacun desdits signaux stockés comprend une pluralité de composants, chaque composant tel étant associé à un desdits transducteurs, ladite étape d'actionnement de chaque émetteur dans ledit ensemble d'émetteurs étant exécutée par actionnement de chacun des émetteurs dans ledit ensemble individuellement alors que ledit récepteur est disposé à chacun desdits points de modèle.
